# EUROPEAN PATENT APPLICATION

(11) **EP 1 614 429 A1**
(43) Date of publication of application: **11.01.2006**
(21) Application number: 04727409.7
(22) Date of filing: 14.04.2004
(51) Int. Cl.: A61K 45/00, A61K 31/366, A61K 31/404, A61K 31/4418, A61K 31/40, A61K 31/505, A61K 31/47, A61K 31/22, A61P 9/00, A61P 9/10, C12N 15/00

(54) **LKLF/KLF2 GENE EXPRESSION PROMOTER**

(30) Priority: 17.04.2003 US 463311 P
(71) Applicant: KOWA CO. LTD., Nagoya-shi Aichi 460-8625 (JP); Nissan Chemical Industries, Ltd., Chiyoda-ku, Tokyo 101-0054 (JP)
(72) Inventor: KODAMA, Tatsuhiko, 1540002 (JP); MORIKAWA, Shigeru, 1710033 (JP)
(74) Representative: Hartz, Nikolai
(86) International application number: PCT/JP2004/005316
(87) International publication number: WO 2004/091660

(57) **Abstract**

Expression of LKLF/KLF2 gene can be promoted by administering, as an active ingredient, an effective amount of a substance capable of inhibiting the mevalonic acid metabolic pathway.

## Description

### Technical Field

This invention relates to lung Kruppel-like factor/KLF2 (hereinafter abbreviated as "LKLF/KLF2") gene expression promoters, which are useful for the treatment and/or decrudescence of diseases associated with blood vessels, for example, diabetes, effort angina, unstable angina, angina pectoris decubitus, myocardial infarction, atherosclerosis, hemoendothelial function disorder, post-PTCA restenosis, hypertensivity pneumonitis, interstitial pneumonia, airway constriction, airway obstruction, eyeground bleeding (retinal vein occlusion, vitreous floaters, etc.), cerebrovascular dementia, cerebral infarction, cerebral hemorrhage, subarachnoid hemorrhage, hemorrhoid, and the like.

### Background Art

LKLF/KLF2 is a transcription regulatory factor protein having structures of proline-rich repeats, an activation domain, a nuclear localization signal and a zinc finger domain [Kozyrev S.V., et al., FEBS Lett., 448(1), 149-52, April 1, 1999]. As an effect of LKLF/KLF2, LKLF/KLF2 is known inter *alia* to be important for hemocyte differentiation [Kuo C.T., et al., Genes Dev., 11(22), 2996-3006, 1997; Anderson K.P. et al., Mol. Cell Biol., 15(11), 5957-65, 1995], to be an important signal transduction factor between vascular endothelial cells and smooth muscle cells [Monajemi H., et al., Thromb. Haemost., 86 (1), 404-12, 2001], to decrease proliferation of T cells, to reduce cell size and protein synthesis and to decrease surface expression of activation markers [BuckleyA.F., et al., Nat. Immunol., 2 (8) , 698-704, 2001], and further, to be essential for blood vessel stabilization [Kuo C.T., etal., Genes Dev., 11 (22), 2996-3006, 1997].

On the other hand, initial lesions of atherosclerosis are known to frequently occur at vessel bifurcations and curvatures where blood flow varies significantly. As a cause of their occurrence, shear stress of the blood flow on the vascular endothelium is considered to play a role. According to recent reports, however, it is pointed out that LKLF/KLF2 is expressed from vascular endothelial cells under shear stress [Dekker R. J. , et al. , Blood, 100(5), 1689-98, 2002] and that LKLF/KLF2 suppressively takes part in the occurrence of atherosclerosis [Karin Arkenbout E., et al., Thromb. Haemost., 89(3), 522-9, 2003]. As appreciated from the foregoing, LKLF/KLF2 which is expressed from vascular endothelial cells is presumed to suppressively act on lesions associated with blood vessels.

Promotion of LKLF/KLF2 gene expression is expected to achieve decrudescence or treatment of diseases associatedwith blood vessels which is ledby arterial sclerosis etc. However, no substance which is able to promote the expression of LKLF/KLF2 gene, including physiological ones, has been known so far.

### Disclosure of the Invention

An object of the present invention is to provide an LKLF/KLF2 gene expressionpromoter effective for the treatment and/or decrudescence of diseases associated with blood vessels, for example, diabetes, effort angina, unstable angina, angina pectoris decubitus, myocardial infarction, atherosclerosis, hemoendothelial function disorder, post-PTCA restenosis, hypertensivity pneumonitis, interstitial pneumonia, airway constriction, airway obstruction, eyeground bleeding (retinal vein occlusion, vitreous floaters, etc.), cerebrovascular dementia, cerebral infarction, cerebral hemorrhage, subarachnoid hemorrhage, hemorrhoid, and the like.

Using a culturedhuman cell system, the present inventors have searched for substances which might affect the expression of LKLF/KLF2 gene. As a result, it has been found that substances capable of inhibiting the mevalonic acid metabolic pathway have activities to promote the expression of LKLF/KLF2 gene, leading to the completion of the present invention.

The present inventors have also found that among the substances capable of inhibiting the mevalonic acid metabolic pathway, compounds represented by the below-described formula (1) which are known as HMG-CoA reductase inhibitors and their lactone derivatives and salts of the compounds and derivatives, especially pitavastatin calcium has activities to promote the expression of LKLF/KLF2 gene.

The present inventors have also found that among the substances capable of inhibiting the mevalonic acid metabolic pathway, farnesyltransferase inhibitors such as FTI-276, geranylgeranyltransferase I inhibitors such as GGTI-286 and glycosyltransferases such as TcdB *(Clostridium difficile* Toxin B) are also in possession of activities to promote the expression of LKLF/KLF2 gene.

Described specifically, the present invention provides an LKLF/KLF2 gene expression promoter, which comprises as an active ingredient a substance capable of inhibiting the mevalonic acid metabolic pathway.

The present invention also provides an LKLF/KLF2 gene expression promoter, which comprises as an active ingredient a compound represented by the following formula (1): 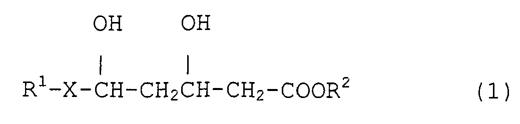
wherein R¹ represents an organic group, X represents -CH₂CH₂- or -CH=CH-, and R² represents a hydrogen atom or an alkyl group, or a lactone derivative thereof, or a salt thereof.

The present invention also provides an LKLF/KLF2 gene expression promoter, which comprises as an active ingredient a farnesyltransferase inhibitor.

The present invention also provides an LKLF/KLF2 gene expression promoter, which comprises as an active ingredient a geranylgeranyltransferase I inhibitor.

The present invention also provides an LKLF/KLF2 gene expression promoter, which comprises as an active ingredient a glucosyltransferase.

The present invention further provides use of a substance capable of inhibiting the mevalonic acid metabolic pathway as an active ingredient for the production of an LKLF/KLF2 gene expression promoter.

The present invention further provides use of a compound, which is representedby the formula (1) , or a lactone derivative thereof, or a salt thereof as an active ingredient for the production of an LKLF/KLF2 gene expression promoter.

The present invention further provides use of a farnesyltransferase inhibitor as an active ingredient for the production of an LKLF/KLF2 gene expression promoter.

The present invention further provides use of a geranylgeranyltransferase I inhibitor as an active ingredient for the production of an LKLF/KLF2 gene expression promoter.

The present invention further provides use of a glucosyltransf erase as an active ingredient for the production of an LKLF/KLF2 gene expression promoter.

The present invention still further provides a method for promoting expression of LKLF/KLF2 gene, which comprises administering, as an active ingredient, an effective amount of a substance capable of inhibiting the mevalonic acid metabolic pathway.

The present invention still further provides a method for promoting expression of LKLF/KLF2 gene, which comprises administering, as an active ingredient, an effective amount of a compound, which is represented by the formula (1), or a lactone derivative thereof, or a salt thereof.

The present invention still further provides a method for promoting expression of LKLF/KLF2 gene, which comprises administering, as an active ingredient, an effective amount of a farnesyltransferase inhibitor.

The present invention still further provides a method for promoting expression of LKLF/KLF2 gene, which comprises administering, as an active ingredient, an effective amount of a geranylgeranyltransferase I inhibitor.

The present invention still further provides a method for promoting expression of LKLF/KLF2 gene, which comprises administering, as an active ingredient, an effective amount of a glucosyltransferase.

According to the present invention, it is possible to provide a method for promoting expression of LKLF/KLF2 gene. This method is effective for the treatment and/or decrudescence of diseases associated with blood vessels, for example, diabetes, effort angina, unstable angina, angina pectoris decubitus, myocardial infarction, atherosclerosis, hemoendothelial function disorder, post-PTCA restenosis, hypertensivity pneumonitis, interstitial pneumonia, airway constriction, airway obstruction, eyeground bleeding (retinal vein occlusion, vitreous floaters, etc.), cerebrovascular dementia, cerebral infarction, cerebral hemorrhage, subarachnoid hemorrhage, hemorrhoid, and the like.

### Brief Description of the Drawings

FIG. 1 is a diagram showing expression levels of LKLF/KLF2 gene in the presence of pitavastatin calcium;
FIG. 2 is a diagram illustrating effects of mevalonic acid and its various metabolites on the expression level of LKLF/KLF2 gene in the presence of pitavastatin calcium;
FIG. 3 is a diagram depicting expression levels of LKLF/KLF2 gene in the presence of substances capable of inhibiting the mevalonic acid metabolic pathway; and
FIG. 4 is a diagram showing the concentration dependencies of substances, which are capable of inhibiting the mevalonic acid metabolic pathway, for the expression level of LKLF/KLF2 gene.

### Best Modes for Carrying Out the Invention

The term "substance capable of inhibiting the mevalonic acid metabolic pathway", which is useful in the present invention, includes therein substances capable of inhibiting any metabolic pathways relating to the metabolism of mevalonic acid. In addition to the main pathway of acetyl-CoA → acetoacetyl-CoA → HMG-CoA → mevalonic acid → mevalonyl-5-phosphate → mevalonyl-5-pyrophosphate → isopentenyl pyrophosphate → geranyl pyrophosphate → farnesyl pyrophosphate → squalene → squalene epoxide → lanosterol → (desmosterol or lathosterol) → cholesterol, thesemetabolic pathways also include the pathway of (isopentenyl pyrophosphate or farnesyl pyrophosphate) → geranylgeranyl pyrophosphate → geranylgeranylated proteins (for example, the Rho protein family such as RhoAB, Rapla, Rac and Cdc42, etc.) and the pathway of farnesyl pyrophosphate → farnesylated proteins (for example, Ras, RhoB, etc.) . Among these, HMG-CoA reductase inhibitors, farnesyltransferase inhibitors, geranylgeranyltransferase I inhibitors and glucosyltransferases are preferred. More preferred are HMG-CoA reductase inhibitors, with pitavastatin and salts thereof being most preferred.

Compounds represented by the formula (1) , their lactone derivatives and salts of these compounds and lactone derivatives, all of which are usable in the present invention, are known as HMG-CoA reductase inhibitors useful as hyperlipidemia therapeutics.

The organic group represented by R¹ in the compound represented by the formula (1) may preferably be a substituted or unsubstituted organic group having a cyclic structure.

Examples of the organic group having the cyclic structure include indolyl, indenyl, pyridyl, pyrrolopyridyl, pyrazolopyridyl, thienopyridyl, pyrimidyl, pyrazolyl, pyrrolyl, imidazolyl, indolidyl, quinolyl, naphthyl, hexahydronaphthyl, cyclohexyl, phenylsilylphenyl, phenylthienyl and phenylfuryl groups, with hexahydronaphthyl, indolyl, pyridyl, pyrimidyl, pyrrolyl and quinolyl groups being particularly preferred.

Examples of substituent groups, which may substitute on these organic groups having the cyclic structures, include hydroxyl group, linear, branched or cyclic alkyl groups, alkyloxyalkyl groups, alkylcarbonyloxy groups, alkyl-substituted amino groups, substituted alkylsulfonylamino groups, substituted phenylsulfonylamino groups, carbamoyl group which may be substituted by one or two alkyl or phenyl groups, halophenyl groups, alkylphenyl groups, alkoxyphenyl groups, phenyl group, and oxo group.

Among these substituents which may substitute on these organic groups having the cyclic structures, preferred are linear, branched or cyclic C₁₋₆ alkyl groups, C₂₋₇ alkyloxyalkyl groups, C₁₋₄ acyloxy groups, C₁₋₄ alkyl-substituted amino groups, C₁₋₄ alkyl-substituted C₁₋₄ alkylsulfonylamino groups, C₁₋₄ alkyl-substituted phenylsulfonylamino groups, C₁₋₄ alkyl-substituted carbamoyl groups, phenyl-substituted carbamoyl groups, fluorophenyl groups, bromophenyl groups, iodophenyl groups, methylphenyl groups, ethylphenyl groups, methoxyphenyl groups, ethoxyphenyl groups and phenyl group, with isopropyl, cyclopropyl and p-fluorophenyl groups being particularly preferred.

Examples of the alkyl group represented by R² may include a linear, branched or cyclic alkyl group having 1-6 carbon atoms.

The lactone derivative can be obtained by subjecting its corresponding compound, which is represented by the formula (1), to lactonization in a manner known *per se* in the art, for example, under acidic conditions.

The salts of the compound represented by the formula (1) and its lactone derivative are physiologically acceptable salts. Examples thereof include alkali metal salts such as the sodium salts and potassium salts, alkaline earth metal salts such as the calcium salts and magnesium salts, organic amine salts such as the phenethylamine salts, and the ammonium salts, with the sodium salts and calcium salts being more preferred.

These compounds are disclosed, for example, in US-A-4,739,073 and EP-A-114,027; EP-A-367,895; US-A-5,001,255, US-A-4,613,610, US-A-4,851,427, US-A-4,755,606, US-A-4,808,607, US-A-4,751,235, US-A, 4, 939, 159, US-A-4, 822, 799, US-A-4,804,679, US-A-4,876,280, US-A-4,829,081, US-A-4,927,851, US-A-4,588,715; F.G. Kathawala, Medical Research Reviews, 11, 121-146 (1991), EP-A-304,063; EP-A-330,057; US-A-5,026,708, US-A-4, 868, 185; EP-A-324, 347; EP-A-300, 278; US-A-5, 013, 749, US-A-5,872,130, US-A-5,856,336, US-A-4,231,938, US-A-4,444,784, US-A-4,346,227, US-A-5,354,772, US-A-5, 273, 995, US-A-5, 177, 080, US-A-3,983,140, JP-B-2,648,897, US-A-5,260,440, Bioorganic & Medicinal Chemistry, 5, 437 (1977), JP-B-2,569,746, EP-B-304,063, and US-A-5, 856, 336.

Preferred examples of the active ingredient in the method according to the present invention for the promotion of expression of LKLF/KLF2 gene include lovastatin (US-A-4,231,938:
(+) - (1S, 3R, 7S, 8S, 8aR) -1, 2, 3, 7, 8, 8a-hexahydro-3, 7-dimethyl -8-[2-[(2R,4R)-tetrahydro-4-hydroxy-6-oxo-2H-pyran-2-yl]e thyl]-1-naphthyl (S)-2-methylbutyrate), simvastatin (US-A-4,444,784:
(+) - (1S, 3R, 7S, 8S, 8aR) -1, 2, 3, 7, 8, 8a-hexahydro-3,7-dimethyl -8-[2-[(2R,4R)-tetrahydro-4-hydroxy-6-oxo-2H-pyran-2-yl]e thyl]-1-naphthyl 2,2-dimethylbutanoate), pravastatin (US-A-4,346,227:
(+)-(3R,5R)-3,5-dihydroxy-7-[(1S,2S,6S,8S,8aR)-6-hydroxy-2-methyl-8-[(S)-2-methylbutyryloxy]-1,2,6,7,8,8a-hexahydr o-1-naphthyl]heptanoic acid), fluvastatin (US-A-5,354,772: (3RS,5SR,6E)-7-[3-(4-fluorophenyl)-1-(1-methylethyl)-1H-i ndol-2-yl]-3,5-dihydroxy-6-heptenoic acid), atorvastatin (US-A-5,273,995:
(3R,5R)-7-[2-(4-fluorophenyl)-5-isopropyl-3-phenyl-4-phen ylcarbamonyl-1H-pyrrol-1-yl]-3,5-dihydroxyheptanoic acid), cerivastatin (US-A-5,177,080:
(3R,5S) -erythro- (E) -7-[4-(4-fluorophenyl)-2,6-diisopropyl -5-methoxymethyl-pyridin-3-yl]-3,5-dihydroxy-6-heptenoic acid), mevastatin (US-A-3,983,140:
(+)-(1S,3R,7S,8S,8aR)-1,2,3,7,8,8a-hexahydro-7-methyl-8-[ 2-[(2R,4R)-tetrahydro-4-hydroxy-6-oxo-2H-pyran-2-yl]ethyl ]-1-naphthyl(S)-2-methylbutyrate), rosuvastatin (US-A-5, 260, 440, JP-B-2, 648, 897 :
7-[4-(4-fluorophenyl)-6-isopropyl-2-(N-methyl-N-methanesu lfonylaminopyrimidin)-5-yl]-(3R,5S)-dihydroxy-(E)-6-hepte noic acid), and pitavastatin (US-A-5,856,336, JP-B-2, 569, 746:
(3R,5S,6E)-7-[2-cyclopropyl-4-(4-fluorophenyl)-3-quinolyl ]-3,5-dihydroxy-6-heptenoic acid, and their salts. In particular, pitavastatin and its salts are preferred.

Examples of the farnesyltransferase inhibitor useful in the present invention include FTI-276, FTI-277, FPT Inhibitor I, FPT Inhibitor II, FPT Inhibitor III, FTase Inhibitor I, FTase Inhibitor II, FTase Inhibitor III, and FTase Inhibitor IV (all, products of Calbiochem of EMD Biosciences, Inc.).

Examples of the geranylgeranyltransferase I inhibitor useful in the present invention include GGTI-286, GGTI-287, GGTI-297, GGTI-298, GGTI-2133, and GGTI-2147 (all, products of Calbiochem of EMD Biosciences, Inc.).

Examples of the glucosyltransferase useful in the present invention include TcdB *(Clostridium difficile* Toxin B) , *Clostridium sordellii* haemorrhagic toxin*,* and *Clostridium sordellii* lethal toxin (all, products of Sigma-Aldrich Co.) .

The substance capable of inhibiting the mevalonic acid metabolic pathway in the present invention, especially the compound (1), its lactone derivative, the salt of the compound or lactone derivative, the farnesyltransferase inhibitor, the geranylgeranyltransferase I inhibitor, the glucosyltransferase or the like significantly promotes the expression of mRNA of LKLF/KLF2 in human cells, and therefore, is useful in the method of the present invention for promoting the expression of LKLF/KLF2 gene and is useful for the treatment of diseases in the onset of which LKLF/KLF2 takes part, especially the treatment of diseases associated with blood vessels.

Further, use of the substance capable of inhibiting the mevalonic acid metabolic pathway, especially, the compound (1) , its lactone derivative, the salt of the compound or lactone derivative, the farnesyltransferase inhibitor, the geranylgeranyltransferase I inhibitor, the glucosyltransferase or the like makes it possible *inter alia* to develop experiment systems in which LKLF/KLF2 takes part and to screen novel medicines.

Illustrative administration routes for the substance capable of inhibiting the mevalonic acid metabolic pathway in the present invention, especially the compound (1) or its lactone derivative or the salt of the compound or lactone derivative, the farnesyltransferase inhibitor, the geranylgeranyltransferase I inhibitor, the glucosyltransferase or the like include oral administrations by tablets, capsules, a granule, a powder, a syrup and the like; and parenteral administrations by an intravenous injection, an intramuscular injection, suppositories, an inhalant, a transdermal preparation, an eye drop, a nasal drop and the like.

To formulate medicinal preparations in such various forms as described above, the active ingredient can be used either singly or in combination with one or more of pharmaceutically acceptable excipients, binders, extenders, disintegrants, surfactants, lubricants, dispersants, buffering agents, preservatives, corrigents, perfumes, coating materials, carriers, diluents and the like, as needed.

Of these administration routes, oral administrations are preferred.

Upon formulation of a medicinal preparation for oral administration of the compound (1), for example, it is preferred to adjust the pH in view of the stability of the active ingredient (JP-A-2-0006406, JP-B-2,774,037, WO-A-97/23200, etc.).

The dosage of the active ingredient varies inter alia depending on the weight, age, sex, conditions and the like of each patient. In the case of an adult, however, it is generally preferred to orally or parenterally administer the active ingredient at a daily dosage of from 0.01 to 1, 000 mg, specifically from 0.1 to 100 mg in terms of the compound represented by formula (1) at once or in several portions.

### Examples

The present invention will hereinafter be described in detail based on Examples. It should however be borne in mind that the present invention is not limited to the following Examples.

### Example 1

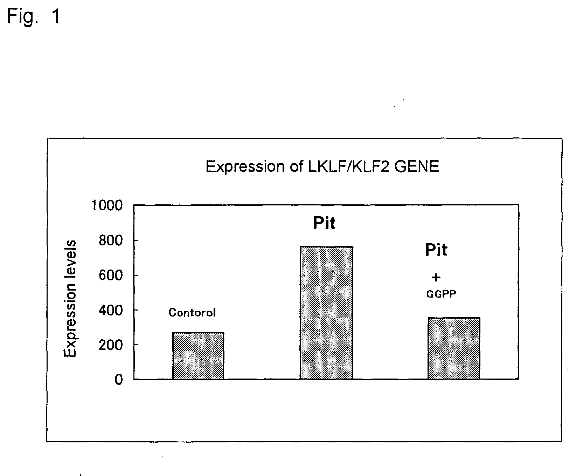

Two days after inoculation of normal human umbilical vein endothelial cells (HUVEC) at 3 x 10⁵ cells/10 cm dish, pitavastatin calcium (indicated as "Pit" in FIG. 1) was added to 1.1 µmol/L. Dimethyl sulfoxide, a solvent for pitavastatin calcium, was added to a control (final concentration: 0.0066 v/v%). Eight hours after the addition, total RNAs were extracted with "ISOGEN" (trade mark, product of NIPPON GENE CO., LTD.). The following procedures were conducted in accordance with the procedures manual of Affymetrix. Inc. Described specifically, following the methods known *per se* in the art, mRNA was isolated from each total RNA obtained above, and based on the mRNA, cDNA was synthesized. Further, biotin-labeled cRNAwas synthesizedby *in vitro* transcription. Subsequent to purification, the biotin-labeled cRNA was subjected to fragmentation by heat treatment to prepare fragmented cRNA for use in a gene expression analysis.

Gene expression analysis method: The fragmented cRNA was poured into "Human Genome Focus Array" (trade name, product of Affymetrix, Inc.), and hybridization was conducted at 45°C for 16 hours. Subsequent to washing, staining with phycoerythrin-labeled streptavidin and biotinylated antistreptavidin antibody was applied, and gene expression information was inputted by "GeneChip™ Scanner" (trade name, manufactured by Hewlett Packard Company). The information was analyzed by "GENECHIP SOFTWARE" (trade name, product of Affymetrix, Inc.) to effect a comparison in expression level.

The results of the measurements are shown in FIG. 1.

The expression of LKLF/KLF2 gene in HUVEC upon elapsed time of 8 hours after the addition of the active ingredient was significantly promoted to 761 in the pitavastatin calcium addition group as opposed to 271 in the control. Further, this effect available from the addition of pitavastatin calcium decreased to 355 by the addition of 10 µmol geranylgeranyl pyrophosphate (GGPP). Involvement of the Rho factor family in the acting mechanism of pitavastatin calcium was suggested accordingly.

### Example 2

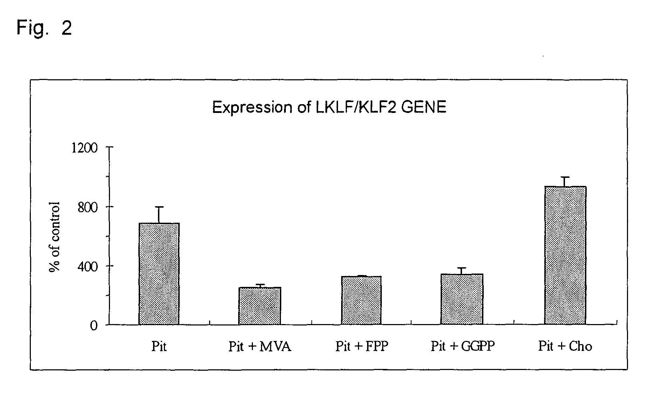

Four days after inoculation of normal human umbilical vein endothelial cells (HUVEC) at 6 x 10⁴ cells/6 well plate, pitavastatin calcium (1 µmol/L; indicated as "Pit" in FIG. 2) was added in combination with mevalonic acid and various metabolites thereof, i.e., mevalonic acid (100 µmol/L; indicated as "MVA" in FIG. 2), farnesyl pyrophosphate (10 µmol/L; indicated as "FPP" in FIG. 2), geranylgeranyl pyrophosphate (10 µmol/L; indicated as "GGPP" in FIG. 2) and cholesterol (10 µmol/L; indicated as "Cho" in FIG. 2), respectively. A group added with no drug (final concentration: 0.1% DMSO) and a group added with pitavastatin calcium alone (1 µmol/L) were also provided as a control and a comparative group, respectively. Eight hours after the addition of the individual drugs, total RNAs were extracted with "ISOGEN" (trade mark, product of NIPPON GENE CO., LTD.). From each total RNA so obtained, DNA was synthesized by using a reverse transcriptase. Using the following primers, the DNA was analyzed by "ABI PRISM 7000 Sequence Detection System" (manufactured by Applied Biosystems JAPAN Ltd.). Kruppel-like factor 2 (KLF2): the forward primer (TCTCTCCCACCGGGTCTACAC: SEQ ID NO: 1); the reverse primer (GCAGACAGTACAAATTAAGGCCCTTA: SEQ IDNO: 2) and the TaqManprobe (AGAGGATCGAGGCTTGTGATGCCTTGT: SEQ ID NO: 3). Internal standard: the forward primer (GCTGGAAGTACCAGGCAGTGA: SEQ ID NO: 4); the reverse primer (TCCGGTAGTGGATCTTGGCTTT: SEQ ID NO: 5) and the TaqMan probe (TCTTTCCTCTTCTCCTCCAGGGTGGCT: SEQ ID NO: 6).

The results are shown in FIG. 2. In the group added with pitavastatin calcium alone, the expression of LKLF/KLF2 gene was observed to have increased by 693% in comparison with the group added with no drug. That increase was reduced by the addition of mevalonic acid, farnesyl pyrophosphate or geranylgeranyl pyrophosphate, but was not affected by cholesterol. Accordingly, the promotion of the expression of LKLF/KLF2 gene by pitavastatin calcium has been found to be reduced by intermediate products other than the final product, cholesterol,in themevalonic acid metabolic pathway.

### Example 3

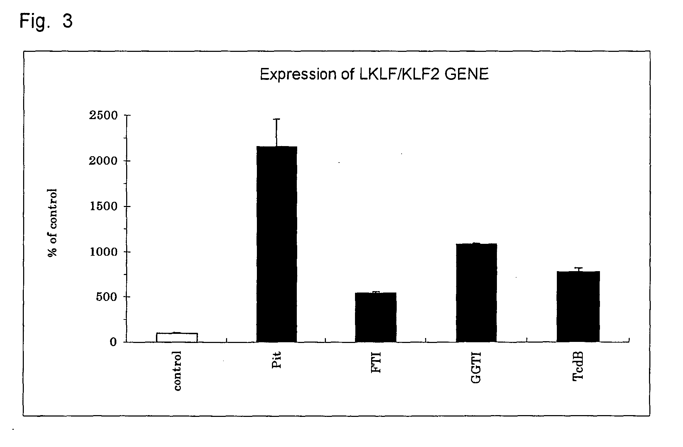

Four days after inoculation of normal human umbilical vein endothelial cells (HUVEC) at 6 x 10⁴ cells/6 well plate, drugs of pitavastatin calcium (1 µmol/L; indicated as "Pit" in FIG. 3), FTI-276 (10 µmol/L; indicated as "FTI" in FIG. 3), GGTI-286 (10 µmol/L; indicated as "GGTI" in FIG. 3) and *Clostridium difficile* Toxin B (50 ng/mL, indicated as "TcdB" in FIG. 3) and as a control, DMSO (final concentration: 0.1%) were added, respectively. Twenty-four hours after the addition, total RNAs were extracted with "ISOGEN" (trade mark, product of NIPPON GENE CO., LTD.). Their measurements were conducted in a similar manner as in Example 2.

The results are shown in FIG. 3. The drugs, which inhibit the mevalonic acid metabolic pathway, each exhibited a strong expression promoting effect as much as 544% to 2, 157% in the expression of LKLF/KLF2 gene compared with the control.

### Example 4

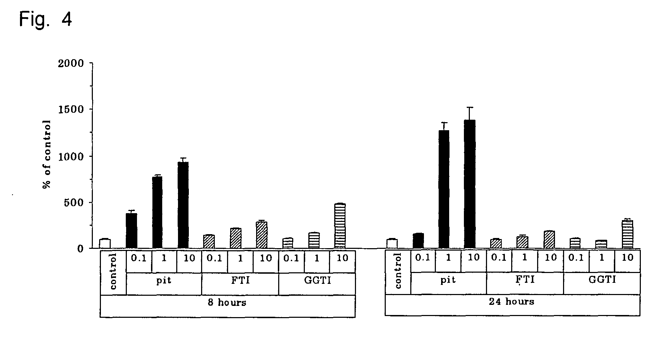

Four days after inoculation of normal human umbilical vein endothelial cells (HUVEC) at 6 x 10⁴ cells/6 well plate, drugs of pitavastatin calcium (0.1, 1, 10 µmol/L; indicated as "Pit" in FIG. 4), FTI-276 (0.1, 1, 10 µmol/L; indicated as "FTI" in FIG. 4), GGTI-286 (0.1, 1, 10 µmol/L; indicated as "GGTI" in FIG. 4) and as a control, DMSO (final concentration: 0. 1%) were added, respectively. Eight hours and 24 hours after the addition, total RNAs were extracted with "ISOGEN" (trade mark, product of NIPPON GENE CO., LTD.). Their measurements were conducted in a similar manner as in Example 2.

As shown in FIG. 4, the drugs, which inhibit the mevalonic acid metabolic pathway, were each found to have concentration-dependant, promoting effect for the expression of LKLF/KLF2 gene.

## Claims

1. An LKLF/KLF2 gene expression promoter comprising, as an active ingredient, a substance capable of inhibiting the mevalonic acid metabolic pathway.

2. The promoter of claim 1, wherein said substance capable of inhibiting the mevalonic acid metabolic pathway is a compound represented by the following formula (1): 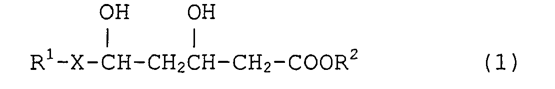
wherein R¹ represents an organic group, X represents -CH₂CH₂- or -CH=CH-, and R² represents a hydrogen atom or an alkyl group, or a lactone derivative thereof, or a salt thereof.

3. The promoter of claim 2, wherein R¹ is a substituted or unsubstituted indolyl, indenyl, pyridyl, pyrrolopyridyl, pyrazolopyridyl, thienopyridyl, pyrimidyl, pyrazolyl, pyrrolyl, imidazolyl, indolidyl, quinolyl, naphthyl, hexahydronaphthyl, cyclohexyl, phenylsilylphenyl, phenylthienyl or phenylfuryl group.

4. The promoter of claim 2, wherein said active ingredient is lovastatin, pravastatin, simvastatin, fluvastatin, cerivastatin, atorvastatin, rosuvastatin, mevastatin or pitavastatin, or a salt thereof.

5. The promoter of claim 2, wherein said active ingredient is pitavastatin or a salt thereof.

6. The promoter of claim 1, wherein said substance capable of inhibiting the mevalonic acid metabolic pathway is a farnesyltransferase inhibitor.

7. The promoter of claim 1, wherein said substance capable of inhibiting the mevalonic acid metabolic pathway is a geranylgeranyltransferase I inhibitor.

8. The promoter of claim 1, wherein said substance capable of inhibiting the mevalonic acid metabolic pathway is a glucosyltransferase.

9. Use of a substance capable of inhibiting the mevalonic acid metabolic pathway as an active ingredient for the production of an LKLF/KLF2 gene expression promoter.

10. Use of a compound, which is represented by the following formula (1): 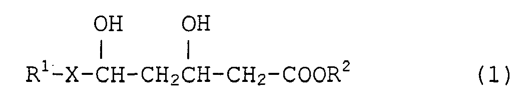
wherein R¹ represents an organic group, X represents -CH₂CH₂- or -CH=CH-, and R² represents a hydrogen atom or an alkyl group, or a lactone derivative thereof, or a salt thereof, as an active ingredient for the production of an LKLF/KLF2 gene expression promoter.

11. The use of claim 10, wherein R¹ is a substituted or unsubstituted indolyl, indenyl, pyridyl, pyrrolopyridyl, pyrazolopyridyl, thienopyridyl, pyrimidyl, pyrazolyl, pyrrolyl, imidazolyl, indolidyl, quinolyl, naphthyl, hexahydronaphthyl, cyclohexyl, phenylsilylphenyl, phenylthienyl or phenylfuryl group.

12. The use of claim 10, wherein said active ingredient is lovastatin, pravastatin, simvastatin, fluvastatin, cerivastatin, atorvastatin, rosuvastatin, mevastatin or pitavastatin, or a salt thereof.

13. The use of claim 10, wherein said active ingredient is pitavastatin or salt thereof.

14. Use of a farnesyltransferase inhibitor as an active ingredient for the production of an LKLF/KLF2 gene expression promoter.

15. Use of a geranylgeranyltransferase I inhibitor as an active ingredient for the production of an LKLF/KLF2 gene expression promoter.

16. Use of a glucosyltransferase as an active ingredient for the production of an LKLF/KLF2 gene expression promoter.

17. Amethod for promoting expression of LKLF/KLF2 gene, which comprises administering, as an active ingredient, an effective amount of a substance capable of inhibiting the mevalonic acid metabolic pathway.

18. The method of claim 17, wherein said substance capable of inhibiting the mevalonic acid metabolic pathway is a compound represented by the following formula (1): 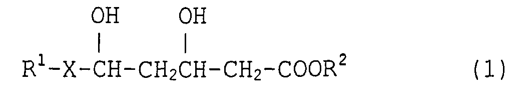
wherein R¹ represents an organic group, X represents -CH₂CH₂- or -CH=CH-, and R² represents a hydrogen atom or an alkyl group, or a lactone derivative thereof, or a salt thereof, as an active ingredient.

19. The method of claim 18, wherein R¹ is a substituted or unsubstituted indolyl, indenyl, pyridyl, pyrrolopyridyl, pyrazolopyridyl, thienopyridyl, pyrimidyl, pyrazolyl, pyrrolyl, imidazolyl, indolidyl, quinolyl, naphthyl, hexahydronaphthyl, cyclohexyl, phenylsilylphenyl, phenylthienyl or phenylfuryl group.

20. The method of claim 18, wherein said active ingredient is lovastatin, pravastatin, simvastatin, fluvastatin, cerivastatin, atorvastatin, rosuvastatin, mevastatin or pitavastatin, or a salt thereof.

21. The method of claim 18, wherein said active ingredient is pitavastatin or salt thereof.

22. The method of claim 17, wherein said substance capable of inhibiting the mevalonic acid metabolic pathway is a farnesyltransferase inhibitor.

23. The method of claim 17, wherein said substance capable of inhibiting the mevalonic acid metabolic pathway is a geranylgeranyltransferase I inhibitor.

24. The method of claim 17, wherein said substance capable of inhibiting the mevalonic acid metabolic pathway is a glucosyltransferase.


SEQUENCE LISTING

<110> KOWA CO., LTD.

<120> An accelerator for the expression of LKLF/KLF2 genes

<130> KW0122

<140>
<141>

<150> US 60/463,311
<151> 2003-04-17

<160> 6

<170> PatentIn Ver. 2.1

<210> 1
<211> 11
<212> DNA
<213> Artificial Sequence

<220>
<223> Description of Artificial Sequence: Designed primer KLF2
      (forward)

<400> 1
tctctcccac cgggtctaca c                                                16

<210> 2
<211> 16
<212> DNA
<213> Artificial Sequence

<220>

<223> Description of Artificial Sequence: Designed primer KLF2
      (Reverse)

<400> 2
gcagacagta caaattaagg ccctta                                          16


<210> 3
<211> 17
<212> DNA
<213> Artificial Sequence

<220>
<223> Description of Artificial Sequence: the TaqMan probe


<400> 3
agaggatcga ggcttgtgat gccttgt                                         17


<210> 4
<211> 11
<212> DNA
<213> Artificial Sequence

<220>
<223> Description of Artificial Sequence: Designed primer KLF2
      (forward)

<400> 4
gctggaagta ccaggcagtg a                                               11


<210> 5
<211> 12
<212> DNA
<213> Artificial Sequence

<220>
<223> Description of Artificial Sequence: Designed primer KLF2
      (Reverse)

<400> 5
tccggtagtg gatcttggct tt                                                12

<210> 6
<211> 17
<212> DNA
<213> Artificial Sequence

<220>
<223> Description of Artificial Sequence: the TaqMan probe

<400> 6
tctttcctct tctcctccag ggtggct                                           17